(19) **Europäisches Patentamt**
**European Patent Office**
**Office européen des brevets**

(11) **EP 4 113 117 B1**

(12) # EUROPEAN PATENT SPECIFICATION

(45) Date of publication and mention
of the grant of the patent:
**03.01.2024 Bulletin 2024/01**

(21) Application number: **21182332.3**

(22) Date of filing: **29.06.2021**

(51) International Patent Classification (IPC):
*G01N 33/24* (2006.01)   *E21B 49/00* (2006.01)
*G01N 15/08* (2006.01)   *G01N 23/046* (2018.01)
*G01N 13/00* (2006.01)   *G01N 23/087* (2018.01)
*G01N 23/095* (2018.01)   *G06F 30/27* (2020.01)
*G06F 30/28* (2020.01)   *G06N 20/00* (2019.01)

(52) Cooperative Patent Classification (CPC):
**G01N 23/046; E21B 41/00; E21B 49/00;**
**G01N 15/082; G01N 23/087; G01N 23/095;**
**G01N 33/241; G06F 30/27; G06F 30/28;**
E21B 2200/20; E21B 2200/22; G01N 2015/0846;
G01N 2223/419; G01N 2223/616; G01N 2223/649;

(Cont.)

(54) **SYSTEM AND METHOD FOR CORRELATING OIL DISTRIBUTION DURING DRAINAGE AND IMBIBITION USING MACHINE LEARNING**

SYSTEM UND VERFAHREN ZUR KORRELATION DER ÖLVERTEILUNG WÄHREND DRAINAGE UND IMBIBITION UNTER VERWENDUNG VON MASCHINENLERNEN

SYSTÈME ET PROCÉDÉ DE CORRÉLATION DE LA DISTRIBUTION D'HUILE PENDANT LE DRAINAGE ET L'IMBIBITION UTILISANT L'APPRENTISSAGE MACHINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**04.01.2023 Bulletin 2023/01**

(73) Proprietor: **Abu Dhabi National Oil Company**
**Abu Dhabi (AE)**

(72) Inventors:
- **Ravoof Shaik, Abdul**
  **Abu Dhabi (AE)**
- **AlSumaiti, Ali M.**
  **Abu Dhabi (AE)**
- **Al Shehhi, Budoor**
  **Abu Dhabi (AE)**
- **Masalmeh, Shehadeh**
  **Abu Dhabi (AE)**
- **Bin Amro, Ahmed**
  **Abu Dhabi (AE)**

(74) Representative: **Bardehle Pagenberg**
**Partnerschaft mbB**
**Patentanwälte Rechtsanwälte**
**Prinzregentenplatz 7**
**81675 München (DE)**

(56) References cited:
**US-A1- 2018 121 579**

- **MOTEALLEH SIYAVASH ET AL: "Unified Model of Drainage and Imbibition in 3D Fractionally Wet Porous Media", TRANSPORT IN POROUS MEDIA, SPRINGER NETHERLANDS, DORDRECHT, vol. 99, no. 3, 26 July 2013 (2013-07-26), pages 581-611, XP035337316, ISSN: 0169-3913, DOI: 10.1007/S11242-013-0201-7 [retrieved on 2013-07-26]**
- **JAMSHIDIAN MAJID ET AL: "A novel estimation method for capillary pressure curves based on routine core analysis data using artificial neural networks optimized by Cuckoo algorithm - A case study", FUEL, IPC SIENCE AND TECHNOLOGY PRESS , GUILDFORD, GB, vol. 220, 22 February 2018 (2018-02-22), pages 363-378, XP085369312, ISSN: 0016-2361, DOI: 10.1016/J.FUEL.2018.01.099**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

• **FENWICK DARRYL H ET AL: "Three-dimensional modeling of three phase imbibition and drainage", ADVANCES IN WATER RESOURCES, vol. 21, no. 2, 1 March 1998 (1998-03-01), - 1 March 1998 (1998-03-01), pages 121-143, XP085283363, ISSN: 0309-1708, DOI: 10.1016/S0309-1708(96)00037-1**

(52) Cooperative Patent Classification (CPC): (Cont.)
G06N 20/00

**Description**

FIELD OF THE INVENTION

The invention relates to measurement of fluid flow properties of a porous rock medium, and in particular to predicting a three-dimensional imbibition phase saturation profile for imbibition of the porous rock medium according to claim 8. The invention further relates to a measurement system for predicting a three-dimensional imbibition phase saturation profile for a porous rock medium according to claim 9 and a method for training of the machine learning algorithm for predicting three-dimensional imbibition phase saturation profile according to claim 1.

BACKGROUND OF THE INVENTION

Extracting hydrocarbons in the form of fluids from subsurface rocks in a subsurface involves an understanding and ability to predict a fluid's movement through the subsurface rocks. Fluid flow of the hydrocarbons and water in the subsurface is significantly impacted by rock heterogeneity of the porous subsurface rocks. An understanding of the fluid flow movement of the hydrocarbons inside the heterogeneous porous medium of the subsurface rocks is a relevant factor in field development planning for oil and gas fields. A wide range of lab experiments are therefore conducted on rock samples, such as core plugs extracted from the subsurface rocks, in order to determine properties of the fluid flow within these subsurface rocks.

Special core analysis laboratory data, oftentimes also referred to as "SCAL-data", is used to understand the fluid flow movement inside the rock samples. This SCAL-data includes, for example, capillary pressure and relative permeability curves of the rock samples. These capillary pressure and relative permeability curves are oftentimes obtained from post processing analysis of experimental measurements performed on the rock samples that are extracted from the subsurface for analysis and evaluation. The SCAL-data such as relative permeability curvature and end-point values are direct indicators of the fluid flow of the hydrocarbon and the water inside the porous rock medium of the rock samples in the subsurface.

Linear X-Ray core flooding equipment is used to determine the fluid flow properties of the porous rock medium. This determining of the fluid flow properties includes capturing information on a one-dimensional phase saturation inside the porous rock medium. Even small variations in the capillary pressure characteristics of the porous rock medium can, however, lead to a saturation heterogeneity. These variations in the capillary pressure characteristics impact results for the relative permeability curves derived from the X-Ray core flooding experiment. The one-dimensional phase saturation information determined from the X-Ray core flooding experiments does therefore not suffice to estimate the relative permeability and the capillary pressure in the rock samples, for example in cases where the rock samples show a strong heterogeneity. This heterogeneity of the porous rock medium impacts the flow of the fluids and trapping of the hydrocarbons in the subsurface on a local level and on a field scale. Understanding and predicting these fluid flow properties is relevant for the extraction of the hydrocarbons from the subsurface rocks. The effect of the heterogeneity on the SCAL-data needs to be characterized and incorporated into a field development plan.

Therefore, capturing three-dimensional water saturation profiles enable an understanding of the fluid flow properties within the porous rock medium. Estimating the capillary heterogeneity and relative permeability also requires a knowledge of distribution of the fluid saturations in the porous rock medium. Medical computer tomography (medical-CT) equipment is therefore used for conducting of so-called "core-flooding experiments" aiming at capturing three-dimensional phase saturation information of the core plugs extracted from the porous rock medium. This acquired three-dimensional phase saturation information is then used in numerical reservoir simulation to quantify capillary heterogeneity and relative permeability of the rock medium. However, extracting a three-dimensional phase saturation profile for each fractional flow is challenging, time-consuming, and expensive. There therefore is a strong need for a time-efficient and cost-effective approach to generate three-dimensional phase saturation profiles.

Various approaches are known in the art for simulation of fluid flow properties within the porous rock medium of the subsurface area. International patent application WO 2019/210102 A1 (Dogru; assigned Aramco Services Co., Saudi Arabian Oil Co.) describes a computer implemented method for the reservoir simulation of fluid flow in a producing reservoir. The method is used for improving the convergence of the determination of the pressure distribution within the reservoir. The pressure distribution is then used to determine initial pressures in the reservoir and estimate the fluid flow in the reservoir. The reservoir comprises rock having heterogeneous properties of permeability and porosity. The reservoir is organized into a three-dimensional grid of reservoir cells. The simulation of the reservoir is performed for a sequence of time steps based on fluid pressures from wells in the reservoir. The method and system do not disclose the use of machine learning for the calculation of the fluid flow.

US patent US 10,718,188 B2 (Dinariev et al; assigned to Schlumberger Technology Corp.) relates to the simulation of a portion of a subterranean formation containing a field (oil or gas reservoir). The method and system disclose the construction of a digital model to represent a portion of the field for the purposes of making decisions regarding the development of the field. The computer model represents the physical space of the reservoir by an array of discrete grid blocks, delineated by a grid which may be regular or irregular. Each block in the grid represents a subsurface volume. The array of grid blocks may be two-dimensional or three-dimensional. Values for physical attributes, such as porosity, permeability and liquid or vapor hydrocarbon saturation, may be associated with each grid block. The value of each attribute may vary across the reservoir volume, but the value is applied uniformly throughout the volume of the grid block. As an example, simulations may solve a complex set of non-linear partial differential equations that model the fluid flow in porous media over a sequence of simulation time points. Grid resolution may impact the accuracy of simulation results, such as in a highly heterogeneous reservoir. However, for practical applications, upscaling techniques may be used to capture fine scale phenomena while using relatively coarse grids. In one or more examples in the document, an upscaling technique is used for simulation of fluid hydrocarbons flow through a heterogeneous reservoir to estimate the volumes of fluid hydrocarbons that may be recoverable. In a further example, the upscaling process may be initiated by the results of a set of pore scale models simulations, whereby the pore scale models used in the simulations represent the pore geometry of the reservoir rock. The method and system do not, however, disclose the use of AI and/or machine learning for the calculation of the fluid flow.

US patent US 10,198,535 B2 (Li et al; assigned to ExxonMobil Upstream Research Co.) discloses a method and system for modeling a hydrocarbon reservoir that includes generating a reservoir model. The reservoir model contains a plurality of volume elements. At least one of the volume elements is simulated using a training simulation to obtain a set of training parameters. The training parameters are, for example, state variables and boundary conditions of the sub region. A machine learning algorithm is then used to approximate an inverse operator of a matrix equation that provides a solution to fluid flow through a porous media. The approximation is done based on the set of defined training parameters. The flow in the (hydrocarbon) reservoir can then be simulated using the inverse operator approximated for the at least one volume element. The method also includes generating a data representation of a physical hydrocarbon reservoir that can be generated in a computer-readable medium based, at least in part, on the results of the simulation. The system and method do not, however, offer a solution for identifying a relationship between the oil distribution during drainage and imbibition of the subsurface area. Motealleh Siyavash et al. disclose an unified model for drainage and imbibition in 3D fractionally wet porous media. Jamshidian Majid et al disclose an estimation method for capillary pressure curves based on routine core analysis data using articial neural networks optimized by Cuckoo algorithm. Fenwick Darryl et al. disclose a three-dimensional modeling of three phase imbibition and drainage.

Other relevant prior art is:

Motealleh Siyavash; Ashouripashaki Mandana; Di-Carlo David; Bryant Steven: "Unified Model of Drainage and Imbibition in 3D Fractionally Wet Porous Media" Transport in Porous Media 99 (3) 581-611 (2013);
Jamshidian Majid; Mansouri Zadeh Mostafa; Hadian Mohsen; Moghadasi Ramin; Mohammadzadeh Omid: "A novel estimation method for capillary pressure curves based on routine core analysis data using artificial neural networks optimized by Cuckoo algorithm - A case study" Fuel 220 363-378 (2018); Fenwick Darryl H; Blunt Martin J: "Three-dimensional modeling of three phase imbibition and drainage" Advances in Water Resources 21 (2) 121-143 (1998); US2018/0121579 A1.

SUMMARY OF THE INVENTION

The present document describes in-situ three-dimensional visualization of phase saturation during water displacing oil process similar to that of three-dimensional saturation profile generated by medical-CT based core flooding equipment.

A computer-implemented method for approximating a predicted three-dimensional imbibition phase saturation profile for imbibition of a porous rock medium is described. The approximating of the three-dimensional imbibition phase saturation profile is done using a machine learning algorithm. The method comprises a first step of inputting at least one of a measured three-dimensional drainage phase saturation profile into the trained machine learning algorithm. The method further comprises the additional steps of inputting a derived one-dimensional drainage phase saturation profile into the trained machine learning algorithm and inputting a one-dimensional imbibition phase saturation profile into the trained machine learning algorithm. The method also comprises the further step of approximating the predicted three-dimensional imbibition phase saturation profile using the using the trained machine learning algorithm, wherein the approximating is done using the processor.

The machine learning algorithm for use in the method is trained using a simulated one-dimensional drainage phase saturation profile, a simulated three-dimensional drainage phase saturation profile, a simulated one-dimensional imbibition phase saturation profile, and a simulated three-dimensional imbibition phase saturation profile.

A measurement system for prediction of a three-dimensional imbibition phase saturation profile is also disclosed. The measurement system for the imbibition of a porous rock medium comprises a processor, a memory, and a detection device. The processor is used for executing at least one of a porous media fluid flow simulator and a machine learning algorithm. The memory is electronically connected to the processor and is used for storing items of data. These items of data stored in the memory comprise at least one of an oil-water contact angle, a relative permeability, and a capillary pressure of a target core plug.

The porous media fluid flow simulator is used to generate SCAL-data which is fed to reservoir simulation models for the prediction of long term oil and gas field behavior as part of a field development planning. The detection device of the system comprises at least one of a linear X-Ray scanner and a medical-CT core flooding equipment.

A computer-implemented method for training a machine learning algorithm is also disclosed. The machine learning algorithm is used to approximate a three-dimensional imbibition phase saturation profile for imbibition of a porous rock medium. The method for the training of the machine learning algorithm comprises the steps of deriving a derived one-dimensional drainage phase saturation profile from a measured three-dimensional drainage phase saturation profile, wherein this deriving is done using a processor. The method further comprises calculating a plurality of synthetic values for a relative permeability of the porous medium and a capillary pressure of the porous medium. This calculating is done using the processor and a measured oil-water contact angle in the porous medium. The method also comprises the feeding of a measured three-dimensional drainage phase saturation profile, the derived one-dimensional drainage phase saturation profile, a one-dimensional imbibition phase saturation profile, the measured oil-water contact angle, the calculated synthetic values for the relative permeability, the calculated synthetic values for the capillary pressure, and a rock heterogeneity state into a porous media fluid flow simulator. The method further comprises the step of generating both a simulated three-dimensional drainage phase saturation profile and a simulated three-dimensional imbibition phase saturation profile using the porous media fluid flow simulator. Using the processor, a simulated one-dimensional drainage phase saturation profile is calculated from the simulated three-dimensional drainage phase saturation profile, and a simulated one-dimensional imbibition phase saturation profile is calculated from the simulated three-dimensional imbibition phase saturation profile. The method further comprises training of the machine learning algorithm. The training is done using the simulated one-dimensional drainage phase saturation profile, the simulated three-dimensional drainage phase saturation profile, the simulated one-dimensional imbibition phase saturation profile, and the simulated three-dimensional imbibition phase saturation profile.

The method further comprises measuring a three-dimensional drainage phase saturation profile using an in-situ core flooding monitoring tool comprising a CT-scanner and the measuring of fluid properties of a crude oil.

The method also comprises a determining of a sister plug for the selected core plug and performing an ageing operation. The method also comprises measuring an oil-water contact angle in the porous medium using analytical or experimental approaches.

The method further comprises determining a rock heterogeneity state by scanning a dry core plug using an CT scanner, for example, Medical CT scanner and micro CT scanner.

DESCRIPTION OF THE FIGURES

Fig. 1A shows a measurement system for predicting a three-dimensional imbibition phase saturation profile.

Fig. 1B shows a first part a flow chart describing a method for training of a machine learning algorithm.

Fig. 1C shows a second part of the flow chart describing a method for training of a machine learning algorithm.

Fig. 1D shows a typical view of measured three dimensional saturation profiles using a medical-CT core flooding scanner.

Fig. 1E shows a schematic view of conversion of a three dimensional phase saturation profile to a one dimensional phase saturation profile along a core length.

Fig.2A shows an example of a simulated three-dimensional drainage phase saturation profile.

Fig. 2B shows an example of a simulated one-dimensional drainage phase saturation profile.

Fig. 2C shows an example of a simulated three-dimensional imbibition phase saturation profile.

Fig. 2D shows an example of a simulated one-dimensional imbibition phase saturation profile.

Fig. 3 shows a permeability distribution of a synthetic heterogeneous core plug having different rock types used for training and validation of a machine learning algorithm.

Fig. 4 show a result of a blind test for an exemplary permeability distribution of a synthetic heterogeneous core plug having different rock types.

Fig. 5 shows an exemplary simulated grid block saturation over a predicted grid block saturation for a blind test core plug.

Fig. 6A and Fig. 6B show an exemplary evaluation of the simulated saturation profile compared to a predicted saturation profile of a blind test core plug.

Fig. 7 shows a flow chart describing the method for approximating a predicted three-dimensional imbibition phase saturation profile.

DETAILED DESCRIPTION OF THE INVENTION

The invention will now be described on the basis of the figures. It will be understood that the embodiments and aspects of the invention described herein are only examples and do not limit the protective scope of the claims in any way. The invention is defined by the claims and their equivalents. It will be understood that features of one aspect or embodiment of the invention can be combined with a feature of a different aspect or aspects and/or embodiments of the invention.

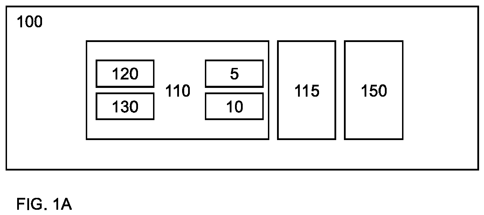

Fig. 1A shows a first view of the measurement system 100 for predicting a three-dimensional imbibition phase saturation profile 35p. The measurement system 100 comprises a processor 110, a memory 115, and a detection device 150. The processor 110 is used for executing at least one of a porous media fluid flow simulator 120 and a machine learning algorithm 130. Items of data generated by the porous media fluid flow simulator 120 are used by reservoir simulation models for the prediction of long-term oil and gas field behavior as part of development planning of the oil and gas field. The memory 115 is electronically connected to the processor 110 and stores items of data. The detection device 150 comprises the linear X-Ray scanner and a medical-CT core flooding equipment. Non-limiting examples of the X-ray scanner are gamma ray core flooding equipment Non-limiting examples of the medical CT core flooding equipment are micro CT core flooding equipment and industrial CT core flooding equipment.

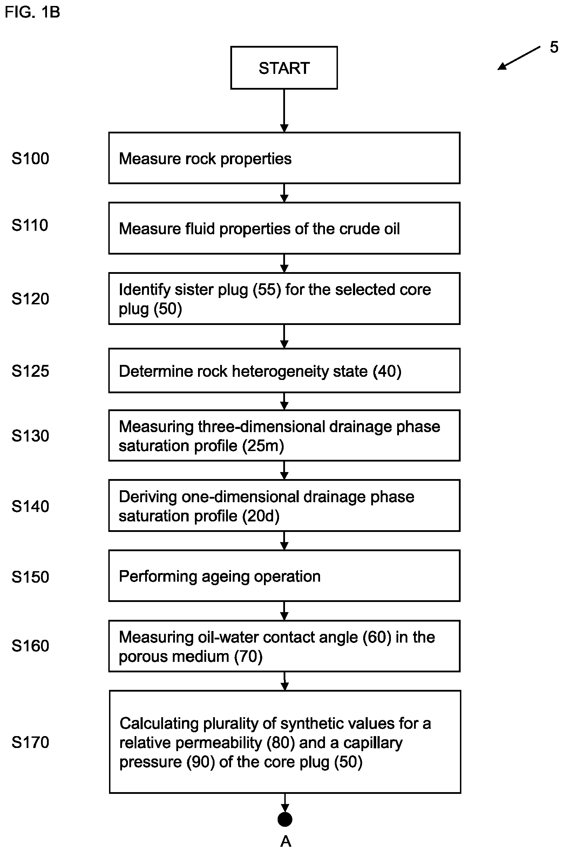

Fig. 1B shows a first part of a flow chart describing a method 5 for training of a machine learning algorithm 130 for predicting a three-dimensional imbibition phase saturation profile 35p for imbibition of a porous rock medium. The method comprises measuring petrophysical properties of a core plug 50 retrieved from a subsurface area containing a significant number of hydrocarbons, such as an oil or gas field. The core plug 50 is a vertically continuous sample extracted from the subsurface area that allows the visual examination of depositional sequences and variations in reservoir character by determining the properties of the subsurface region. The petrophysical properties of the core plug 50 are measured in step S100. These properties include, for example, the porosity, pore size distribution and permeability of the core plug 50 and can indicate evidence of the presence, quantity, distribution, and deliverability of hydrocarbons.

The fluid and chemical properties of the crude oil and brine are determined in step S110 using, for example, a laboratory Pressure-Volume-Temperature cell, also referred to as "PVT cell". Using the PVT cell, the properties of the crude oil such as the molecular weight of the crude oil, the bubble-point pressure of the crude oil, or the oil formation volume factor can be determined.

In step S120, a sister plug 55 is identified for the selected core plug 50. The sister plug 55 is a sample that is extracted from the subsurface area. The sister plug has similar petrophysical properties and heterogeneity compared to the selected core plug 50. This selecting of the sister plug 55 becomes necessary because the analysis of the petrophysical properties of the selected core plug 50 may compromise the results for other laboratory experiments conducted on the selected core plug 50. For example, the measurement of the porosity on the selected core plug 50 may compromise the wettability of the selected core plug 50. The sister plug 55 therefore has to be used for certain laboratory experiments, as will be explained later.

A three-dimensional drainage phase saturation profile 25m for the selected core plug 50 is determined in step S130. The determining of the drainage phase saturation profile 25m is done using a steady state oil displacing water (also called "drainage") core flooding lab experiment with a three-dimensional in-situ saturation monitoring tool (see Jackson et.al, 2018). The one-dimensional drainage phase saturation profile 25d is derived from the measured three-dimensional drainage phase saturation profile 25m using the processor 110. The one-dimensional drainage phase saturation profile 25d is calculated in Step S140 as a mathematical average of the three-dimensional drainage phase saturation at each circular cross section along the core plug 50.

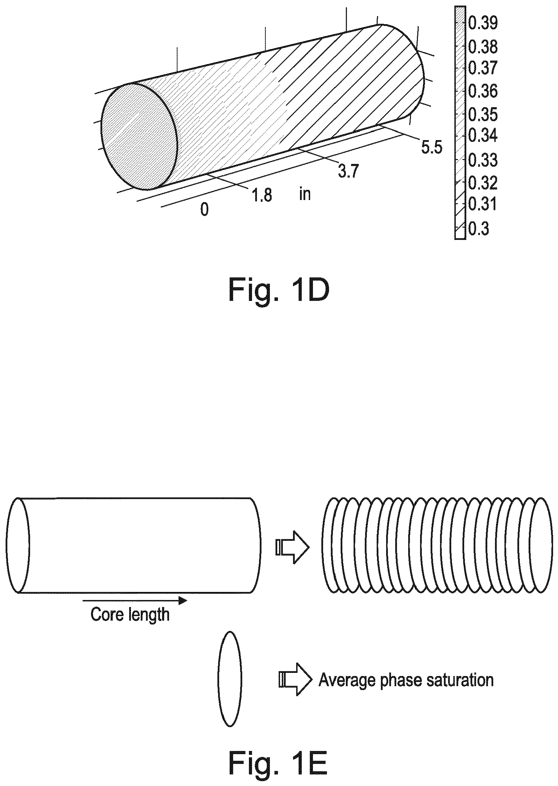

Fig. 1D (see also Hisham et.al, 2021) shows an example of a three-dimensional phase saturation profile, such as the measured three-dimensional drainage phase saturation profile 25m or the predicted three-dimensional imbibition phase saturation profile 35p, that is determined along a length of the core plug 50. These three-dimensional phase saturation profiles are a stack of two-dimensional saturation profiles which are in shape of circular disks as can be seen from Fig. 1E. The mathematical average of these three-dimensional drainage phase saturations at each circular cross section is calculated as one dimensional phase saturation along the length of the core plug 50. This mathematical average is calculated as the sum of all the phase saturations measured at that circular disk and is then divided by a number of locations where phase saturation is measured.

An ageing operation is performed in an 'ageing cell' using representative crude oil on the sister plug 55 in step 5150.

An oil-water contact angle 60 in the sister plug 55 is measured using an analytical or an experimental approach in step S160. The oil-water contact angle 60 in the porous medium 70 describes an angle of intersection of the interface between the two fluids and a porous solid at a solid surface. The oil-water contact angle 60 is geometrically defined as the angle formed by a liquid at the three-phase contact point between where the porous medium 70, the oil and the water intersect. For example, the sister plug 55 is initially cleaned and saturated with water then flooded with oil to reach a state representative of the reservoir. The sister plug 55 is aged using 'ageing cell'. The oil-water contact angle 60 is measured from the solid surface of the porous medium 70 of the sister plug 55 through the oil phase of the porous medium 70 using X-ray micro tomography based equipment such as a microCT scanner or other proven technologies such as a pendent drop method.

This experimental approach for the determining of the oil-water contact angle 60 comprises experimental techniques that are commonly used in the oil and gas industry to measure the contact angle between oil and water (see Andrew et.al, 2014; AlRatrout et.al, 2018; Khishvand et.al, 2017). The oil-water contact angle 60 is determined using micro-CT image data of the sister plug. This micro-CT image data is processed to remove artefacts from the image data. A sub-volume of the micro-CT image data is extracted and re-segmented. This sub-volume is also referred to as "slice". Visibility of edges of phases the fluids and the solids in the sister plug in the slice are enhanced using available image filtering techniques such as a 3D Sobel filter. This enhanced slice of micro-CT image data is also referred to as a "resampled slice". A binary filter is applied to the resampled slice. The oil-water contact angle 60 is measured manually by tracing vectors tangential to the solid surface and the liquid interface seen from the filtered resampled slice of the image data from the sister plug (see Andrew et.al, 2014). This experimental approach for the determining of the oil-water contact angle 60 further comprises estimating of the contact angle using an analytical approach based on the measured drainage capillary pressure and measured imbibition capillary pressures (see Masalmeh, 2001). Fig. 1D shows a typical view of three-dimensional profiles measured using medical-CT core flooding scanner.

A plurality of synthetic values for the relative permeability 80 and a capillary pressure 90 of the selected core plug 50 are calculated in step S170. This calculating is done using mathematical methods such as but not limited to Brooks-Corey, LET and modified Corey models that are available in literature to define the capillary pressure and relative permeability curves. These known models can be used with a plurality of synthetic values for the relative permeability 80 and the capillary pressure 90 of the selected core plug 50. For example, the synthetic relative permeability tables are ensured by varying Corey's exponents $n_{wd}$ and $n_{od}$, saturation endpoints $S_{wc}$ and $K_{ro}^*$, and the fitting parameters $c_{wd}$, $c_{od}$, $a_{wd}$, $a_{od}$ and $b_d$ as described in the modified Corey model (Masalmeh et al, 2007).

The rock heterogeneity state 40 for the selected core plug 50 is determined in step S125 using established approaches, such as determining a porosity variation inside the core plug using dual energy X-ray based CT scanner equipment (see Larmagnat et.al, 2019). The rock heterogeneity state 40 describes a quality of the variation of the rock properties within the core plug 50. This rock heterogeneity state 40 is determined using well established approaches This rock heterogeneity state 40 makes petroleum system modeling, formation evaluation, and reservoir simulation critical to maximizing production from oil and gas reservoirs. For example, a CT scan of the core plug 50 is used to identify the rock heterogeneity state 40.

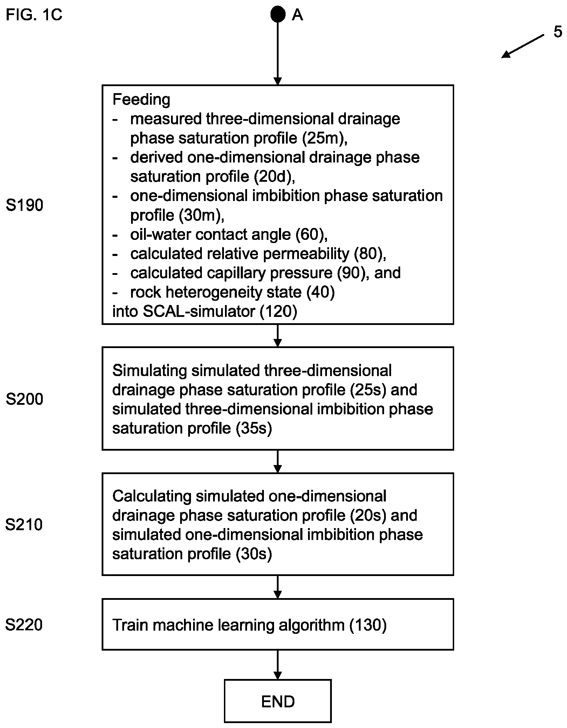

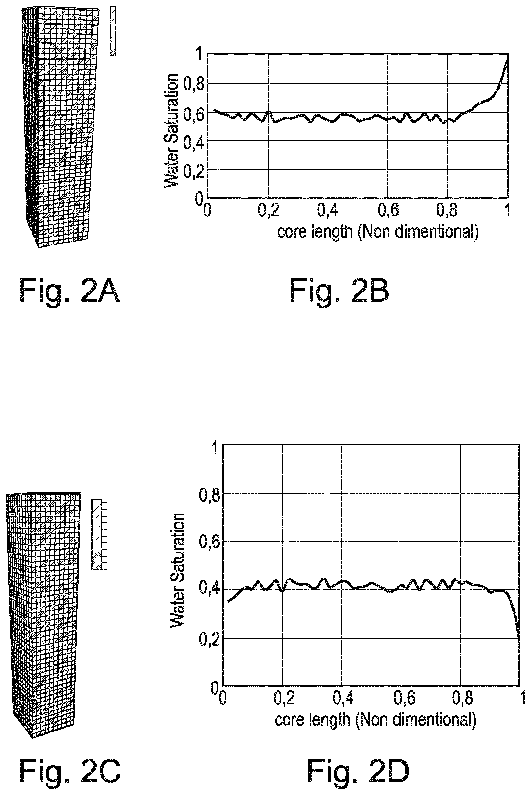

Fig. 1C shows a second part of the flow chart describing the method 5 for training of the machine learning algorithm 130. The measured three-dimensional drainage phase saturation profile 25m, the derived one-dimensional drainage phase saturation profile 20d, a measured one-dimensional imbibition phase saturation profile 30m, the oil-water contact angle 60, the calculated relative permeability 80, the calculated capillary pressure 90, and the rock heterogeneity state 40 are fed into the porous media fluid flow simulator 120 in step S190. The porous media fluid flow simulator 120 can be a proprietary software such as the calculation solutions offered by CMG Computer Modelling Group Ltd. A large number of core flooding experiments are required to determine the simulated three-dimensional drainage phase saturation profile 25s and the simulated three-dimensional imbibition phase saturation profile 35s. Fig. 2A shows an example for the simulated three-dimensional drainage phase saturation profile 25s. Fig. 2C shows an example for the simulated three-dimensional imbibition phase saturation profile 35s. These core flooding experiments are conducted in the porous media fluid flow simulator 120 in step S200.

A simulated one-dimensional drainage phase saturation profile 20s is calculated from the simulated three-dimensional drainage phase saturation profile 25s and a simulated one-dimensional imbibition phase saturation profile 30s is calculated from the simulated three-dimensional imbibition phase saturation profile 35s in step S210 as the mathematical average of the simulated three-dimensional imbibition phase saturation profile 35s at each circular cross section along the core plug 50 using the mathematical average calculation method described in step S140 (see above). Fig. 2B shows an example of the simulated one-dimensional drainage phase saturation profile 20s calculated from the simulated three-dimensional drainage phase saturation profile 25s. Fig. 2D shows an example of the simulated one-dimensional imbibition phase saturation profile 30s calculated from the simulated three-dimensional imbibition phase saturation profile 35s.

The machine learning algorithm 130 is trained using, as a training data set, the plurality of synthetic values for the relative permeability 80 and the capillary pressure 90 of the selected core plug 50 (as determined in step S170), the determined rock heterogeneity state 40 (as determined in step S125), the simulated three-dimensional drainage phase saturation profile (25s), the simulated one-dimensional drainage phase saturation profile 20s, the simulated three-dimensional imbibition phase saturation profile 35s, and the simulated one-dimensional imbibition phase saturation profile 30s. The machine learning algorithm 130 comprises a supervised deep learning algorithm, an unsupervised deep learning algorithm, or a reinforcement deep learning algorithm. The machine learning algorithm 130 comprises proprietary frameworks such as, for example, Tensorflow, Pytorch, or MXNet.

The machine learning algorithm 130 is trained in step S220 using the training data set and a plurality of sets of data for the measured three-dimensional drainage phase saturation profile 25m, the derived one-dimensional drainage phase saturation profile 20d, the measured one-dimensional imbibition phase saturation profile 30m, and the predicted three-dimensional imbibition phase saturation profile 35p which have been classified and form the so-called "ground truth" for the training of the machine learning algorithm 130.

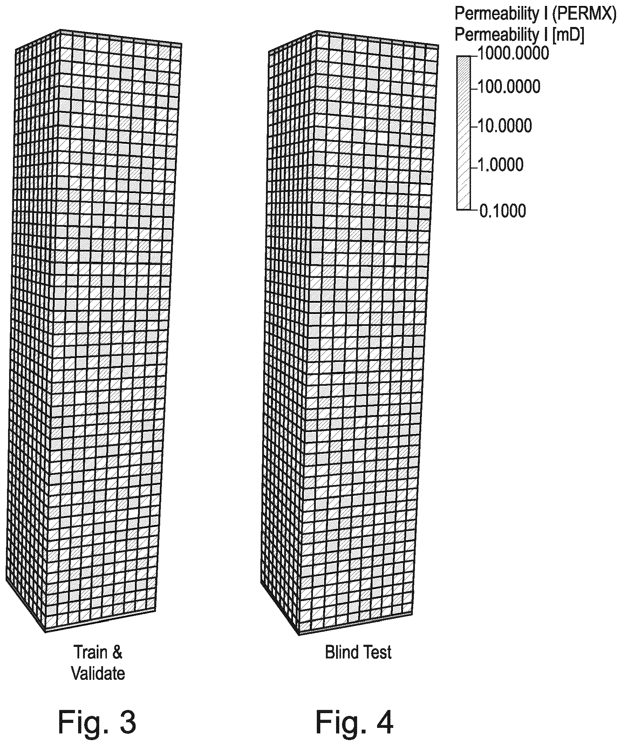

The training of the machine learning algorithm 130 is done by applying the method 5 to a synthetic heterogenous core plug 50 having multiple rock types. The synthetic heterogenous core plug 50 is an exemplary virtual example resembling a physical/real core plug 50. The permeability distribution is determined as the petrophysical properties of the synthetic heterogenous core plug 50, as is described above in step S100. An exemplary result of the determined permeability distribution of the synthetic heterogeneous core plug 50 are shown in Fig. 3 and Fig. 4.

The three-dimensional drainage phase saturation profile 25m for the synthetic heterogenous core plug 50 is determined (as is described above in S130). The determining is done using, for example, ten different fraction flow rates for and calculating three-dimensional drainage phase saturation profile 25m for each of these different fraction flow rates. The one-dimensional drainage phase saturation profile 25d is calculated using the mathematical average (as is described above in Step S140) for each of the ten determined three-dimensional drainage phase saturation profiles 25m.

The steps of performing the ageing operation (see step S150), measuring the oil-water contact angle 60 in the porous medium 70 (see step S160), calculating the plurality of the synthetic values for the relative permeability 80 and the capillary pressure 90 of the synthetic heterogeneous core plug 50 (see step S170) are determined. The measured three-dimensional drainage phase saturation profile 25m, the derived one-dimensional drainage phase saturation profile 20d, the measured one-dimensional imbibition phase saturation profile 30m, the measured oil-water contact angle 60, the calculated synthetic values for the relative permeability 80, the calculated synthetic values for the capillary pressure 90, and the rock heterogeneity state 40 are then input in the porous media fluid flow simulator 120 as inputs. Using these described inputs, the simulated three-dimensional drainage phase saturation profile 25s and the simulated three-dimensional imbibition phase saturation profile 35s are simulated for the different fraction flow rates using the porous media fluid flow simulator 120 as described in step S200 (see above).

The simulated one-dimensional drainage phase saturation profiles 20s and the simulated one-dimensional imbibition phase saturation profiles 30s are calculated from the simulated three-dimensional drainage phase saturation profile 25s and the simulated three-dimensional imbibition phase saturation profile 35s for the different fraction flow rates using the mathematical average (as is described in step S210 above). The machine learning algorithm 130 is trained using the measured three-dimensional drainage phase saturation profile 25m, the derived one-dimensional drainage phase saturation profile 20d, the measured one-dimensional imbibition phase saturation profile 30m, the simulated three-dimensional drainage phase saturation profile 25s, and the simulated three-dimensional imbibition phase saturation profile 35s.

The training of the machine learning algorithm 130 comprises enabling the machine learning algorithm 130 to capture the relationship between the oil distribution during drainage and imbibition of the synthetic heterogeneous core plug 50 (see also step S220 above). During a training phase, an available data set is divided into two subsets, in which one subset is used named as a primary subset and a second subset is named as a secondary subset. The machine learning algorithm 130 provides machine learning results for an analysis request by processing available data set. The results from the machine learning algorithm 130 includes a confidence metric and a prediction made from the input data. For example, the machine learning algorithm provides phase saturation and the confidence metric for each instance of the workload data input into the machine learning algorithm.

Primary data called a "input data" or "ground truth" and secondary data called "output data" is used to train the machine learning algorithm 130. The training of the machine learning algorithm 130 comprises finding and quantifying a relationship between this input data and this output data. The training of the machine learning algorithm 130 therefore comprises comparing the secondary data output by the machine learning algorithm 130 to the primary data for the workload data. If the machine learning algorithm 130 outputs a secondary data being approximately similar to the primary data of the workload data, the machine learning algorithm 130 is rewarded. If the machine learning algorithm 130 outputs a secondary data being unsimilar to the primary data of the workload data, the machine learning algorithm 130 is penalized. Rewarding and penalizing the machine learning algorithm 130 is done for updating of parameters of a model of the machine learning algorithm 130 using a mathematical function such as a "loss function".

The loss function is a mathematical function for evaluating a degree of similarity between the secondary data output by the machine learning algorithm 130 and the primary data. The loss function returns a numerical value (usually a real number) indicating the degree of similarity. If, for example, there is a high degree of similarity between the primary data and the output secondary data, the loss function returns a small numerical value. Similar, if, for example, there is a low degree of similarity between the primary data and the output secondary data, the loss function returns a high numerical value. The loss function therefore indicates how close the secondary data output by the machine learning algorithm 130 is to the primary data. The numerical value of the loss function determines how much the machine learning algorithm 130 should be penalized or rewarded for a current set of model parameters used in the machine learning algorithm 130 in determining the secondary data.

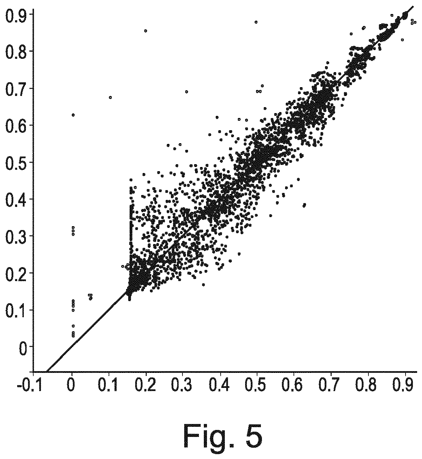

An efficiency of the machine learning algorithm 130 is quantified using the confidence metric, which comprises a percentage, a ratio, a rating on a scale, or another indicator of accuracy, effectiveness, and/or confidence. Examples of the machine learning algorithm 130 include, but are not limited to an artificial neural network, a XGBoost-algorithm, a recurrent neural networks or a combination of these. The trained machine learning algorithm 130 is validated using blind test data generated from different heterogeneous core plugs 50 with a ten percent (10%) error as the ground truth, as can be seen in Fig. 5.

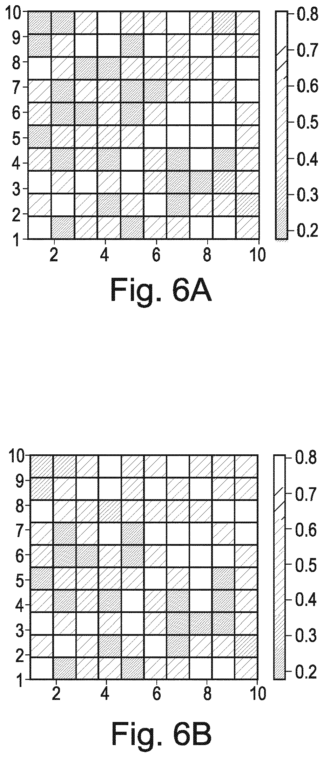

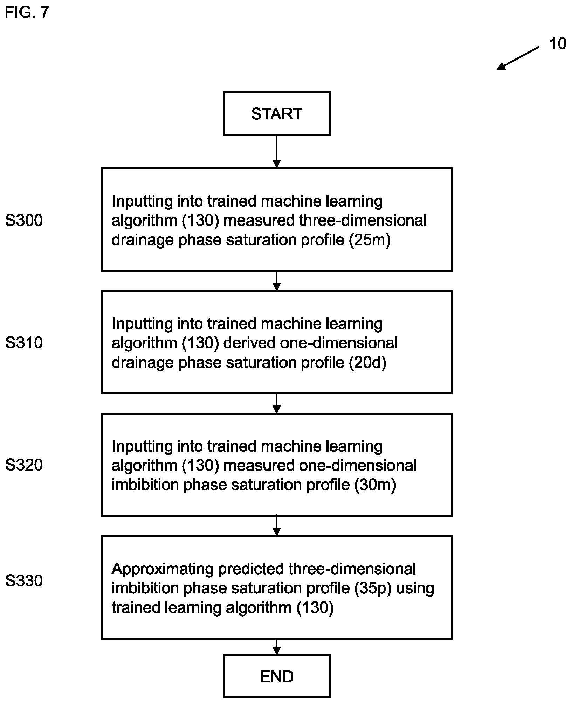

Fig. 6A and Fig. 6B show an exemplary evaluation of the three-dimensional imbibition phase saturation profile. Fig. 6A shows a resulting slice of the three-dimensional imbibition phase saturation profile 35p as simulated by proprietary simulation software at a specific time and position. Fig. 6B shows a slice of the predicted three-dimensional imbibition phase saturation profile 35p at the same specific time and position as Fig 6A using a method 10 described in Fig. 7 and the trained machine learning algorithm 130 (see Fig. 1B and Fig. 1C above). As can be seen from Fig. 6A and Fig. 6B there is a relevant degree of similarity between Fig. 6A and Fig. 6B. This similarity indicates an acceptable match between the simulated three-dimensional imbibition phase saturation profile and the predicted three-dimensional imbibition phase saturation profile 35p.-The root mean square error between individual block saturations in simulated three-dimensional imbibition phase saturation profile and individual block saturations in predicted three-dimensional imbibition phase saturation profile is found to be less than 10%. The accuracy of the prediction can be further improved by using a larger training data set.

Fig. 7 shows a flow chart describing the method 10 for approximating a predicted three-dimensional imbibition phase saturation profile 35p using the trained machine learning algorithm 130. The method 10 comprises inputting the measured three-dimensional drainage phase saturation profile 25m into the trained machine learning algorithm 130 in step S300. The method 10 further comprises inputting the derived one-dimensional drainage phase saturation profile 20d in step S310 and inputting the measured one-dimensional imbibition phase saturation profile 30m in step S320 into the trained machine learning algorithm 130. Using the processor 110 and the trained machine learning algorithm 130, the predicted three-dimensional imbibition phase saturation profile (35p) is approximated in step S330.

Reference numerals

20d    derived one-dimensional drainage phase saturation profile
20s    simulated one-dimensional drainage phase saturation profile
25m    measured three-dimensional drainage phase saturation profile
25s    simulated three-dimensional drainage phase saturation profile
30m    measured one-dimensional imbibition phase saturation profile
30s    simulated one-dimensional imbibition phase saturation profile
30c    calculated one-dimensional imbibition phase saturation profile
35p    predicted three-dimensional imbibition phase saturation profile
35s    simulated three-dimensional imbibition phase saturation profile
40    rock heterogeneity state
50    core plug
55    sister plug
60    oil-water contact angle
70    porous medium
80    relative permeability
90    capillary pressure
100    measurement system
110    processor
115    memory
120    porous media fluid flow simulator
130    machine learning algorithm
150    detection device

**Claims**

1. A computer-implemented method (5) for training a machine learning algorithm (130) for predicting three-dimensional imbibition phase saturation profile (35p) for imbibition of a porous rock medium (70), the method comprising:

   deriving (S140), using a processor (110), a derived one-dimensional drainage phase saturation profile (20d) from a measured three-dimensional drainage phase saturation profile (25m) of a selected core plug (50) of the porous rock medium (70);
   calculating (S170), using the processor (110) and a measured oil-water contact angle (60) in the selected core plug (50) of the porous rock medium (70), a plurality of synthetic values for a relative permeability (80) of the porous rock medium (70) and a capillary pressure (90) of the porous rock medium (70);
   feeding (S190) the measured three-dimensional drainage phase saturation profile (25m), the derived one-dimensional drainage phase saturation profile (20d), a measured one-dimensional imbibition phase saturation profile (30m), the measured oil-water contact angle (60), the calculated synthetic values for the relative permeability (80), the calculated synthetic values for the capillary pressure (90), and a rock heterogeneity state (40) into a porous media fluid flow simulator (120);

simulating (S200), using the porous media fluid flow simulator (120), a simulated three-dimensional drainage phase saturation profile (25s) and a simulated three-dimensional imbibition phase saturation profile (35s);

calculating (S210), using the processor (110), a simulated one-dimensional drainage phase saturation profile (20c) from the simulated three-dimensional drainage phase saturation profile (25s), and a simulated one-dimensional imbibition phase saturation profile (30c) from the simulated three-dimensional imbibition phase saturation profile (35s); and

training (S220), using the simulated one-dimensional drainage phase saturation profile (20s), the simulated three-dimensional drainage phase saturation profile (25s), the simulated one-dimensional imbibition phase saturation profile (30s), and the simulated three-dimensional imbibition phase saturation profile (35s), a machine learning algorithm (130).

2. The computer-implemented method (5) of claim 1, further comprising:
measuring (S130) a three-dimensional drainage phase saturation profile (25m) using an in-situ core flooding monitoring tool comprising a CT-scanner.

3. The computer-implemented method (5) of claims 1 and 2, further comprising:
measuring (S110) fluid properties of a crude oil.

4. The computer-implemented method (5) of claims 1 to 3, further comprising:
identifying (S120) a sister plug (55) for the selected core plug (50).

5. The computer-implemented method (5) of claim 4, further comprising:
performing (S150) an ageing operation on the sister plug (55).

6. The computer-implemented method (5) of claims 1 to 5, further comprising:
measuring (S160) an oil-water contact angle (60) in the porous medium (70) using at least one of analytical or experimental techniques.

7. The computer-implemented method (5) of claims 1 to 6, further comprising:
determining a rock heterogeneity state (40) comprises using a medical CT scanner.

8. A computer-implemented method (10) for predicting a three-dimensional imbibition phase saturation profile (35p) of a porous rock medium (70), the method comprising:

inputting (S300) at least one measured three-dimensional drainage phase saturation profile (25m) of a target core plug (50) into a machine learning algorithm (130) that has been trained according to any one of claims 1-7;
inputting (S310) a derived one-dimensional drainage phase saturation profile(2od) derived from the at least one measured three-dimensional drainage phase saturation profile (25m) into the machine learning algorithm (130);
inputting (S320) at least one of a measured one-dimensional imbibition phase saturation profile (30m) of the target core plug (50) into the machine learning algorithm (130); and
approximating (S330), using the machine learning algorithm (130) executing on a processor (110), the predicted three-dimensional imbibition phase saturation profile (35p) of the porous rock medium (70) based on the input.

9. A measurement system (100) for predicting a three-dimensional imbibition phase saturation profile (35p) for imbibition of a porous rock medium, the system (100) comprising:

a detection device (150) for determining special core analysis laboratory (SCAL) data;
a processor (110), for executing a special core analysis laboratory (SCAL), a porous media fluid flow simulator (120) and a machine learning algorithm (130);
a memory (115), connected to the processor (110), for storing items of SCAL data of a target core plug (50) on an oil-water contact angle (60), and optionally a relative permeability (80), and/or and a capillary pressure (90); and
wherein the machine learning algorithm has been trained according to any one of claims 1-7 and is configured to predict the three-dimensional imbibition phase saturation profile (35p) of the porous rock medium according to claim 8.

10. The measurement system (100) of claim 9, wherein:
the SCAL-data is used in reservoir simulation models for a prediction of oil and gas field behavior.

11. The measurement system (100) of claims 9 and 10, wherein:
the detection device (150) comprises at least one of a linear X-Ray scanner, a Gamma Ray scanner, or a medical-CT core flooding equipment.

**Patentansprüche**

1. Computerimplementiertes Verfahren (5) zum Trainieren eines Maschinenlernalgorithmus (130) zum Vorhersagen eines dreidimensionalen Imbibitionsphasensättigungsprofils (35p) zur Imbibition eines porösen Gesteinsmediums (70), wobei das Verfahren umfasst:

Ableiten (S140), unter Verwendung eines Prozessors (110), eines abgeleiteten eindimensionalen Drainagephasensättigungsprofils (20d) aus einem gemessenen dreidimensionalen Drainagephasensättigungsprofil (25m) eines ausgewählten Kernstopfens (50) des porösen Gesteinsmediums (70);
Berechnen (S170), unter Verwendung des Prozessors (110) und eines gemessenen Öl-Wasser-Kontaktwinkels (60) in dem ausgewählten Kernstopfen (50) des porösen Gesteinsmediums (70), einer Mehrzahl von synthetischen Werten für eine relative Permeabilität (80) des porösen Gesteinsmediums (70) und einen Kapillardruck (90) des porösen Gesteinsmediums (70);
Einspeisen (S190) des gemessenen dreidimensionalen Drainagephasensättigungsprofils (25m), des abgeleiteten eindimensionalen Drainagephasensättigungsprofils (20d), eines gemessenen eindimensionalen Imbibitionsphasensättigungsprofils (30m), des gemessenen Öl-Wasser-Kontaktwinkels (60), der berechneten synthetischen Werte für die relative Permeabilität (80), der berechneten synthetischen Werte für den Kapillardruck (90) und eines Gesteinsheterogenitätszustands (40) in einen Strömungssimulator (120) für poröse Medien;
Simulieren (S200), unter Verwendung des Strömungssimulators (120) für poröse Medien, eines simulierten dreidimensionalen Drainagephasensättigungsprofils (25s) und eines simulierten dreidimensionalen Imbibitionsphasensättigungsprofils (35s);
Berechnen (S210), unter Verwendung des Prozessors (110), eines simulierten eindimensionalen Drainagephasensättigungsprofils (20c) aus dem simulierten dreidimensionalen Drainagephasensättigungsprofil (25s) und eines simulierten eindimensionalen Imbibitionsphasensättigungsprofils (30c) aus dem simulierten dreidimensionalen Imbibitionsphasensättigungsprofil (35s); und
Trainieren (S220), unter Verwendung des simulierten eindimensionalen Drainagephasensättigungsprofils (20s), des simulierten dreidimensionalen Drainagephasensättigungsprofils (25s), des simulierten eindimensionalen Imbibitionsphasensättigungsprofils (30s) und des simulierten dreidimensionalen Imbibitionsphasensättigungsprofils (35s), eines Maschinenlernalgorithmus (130).

2. Computerimplementiertes Verfahren (5) nach Anspruch 1, ferner umfassend:
Messen (S130) eines dreidimensionalen Drainagephasensättigungsprofils (25m) unter Verwendung eines In-situ-Kernflutungsüberwachungswerkzeugs, das einen CT-Scanner umfasst.

3. Computerimplementiertes Verfahren (5) nach Anspruch 1 und 2, ferner umfassend:
Messen (S110) von Fluideigenschaften eines Rohöls.

4. Computerimplementiertes Verfahren (5) nach Anspruch 1 bis 3, ferner umfassend:
Identifizieren (S120) eines Schwesterstopfens (55) für den ausgewählten Kernstopfen (50).

5. Computerimplementiertes Verfahren (5) nach Anspruch 4, ferner umfassend:
Durchführen (S150) eines Alterungsvorgangs an dem Schwesterstopfen (55).

6. Computerimplementiertes Verfahren (5) nach Anspruch 1 bis 5, ferner umfassend:
Messen (S160) eines Öl-Wasser-Kontaktwinkels (60) in dem porösen Medium (70) unter Verwendung von analytischen und/oder experimentellen Techniken.

7. Computerimplementiertes Verfahren (5) nach Anspruch 1 bis 6, ferner umfassend:
Bestimmen eines Gesteinsheterogenitätszustands (40) umfasst Verwenden eines medizinischen CT-Scanners.

8. Computerimplementiertes Verfahren (10) zum Vorhersagen eines dreidimensionalen Imbibitionsphasensättigungsprofils (35p) eines porösen Gesteinsmediums (70), wobei das Verfahren umfasst:

Eingeben (S300) mindestens eines gemessenen dreidimensionalen Drainagephasensättigungsprofils (25m) eines Zielkernstopfens (50) in einen Maschinenlernalgorithmus (130), der nach einem der Ansprüche 1-7 trainiert wurde;
Eingeben (S310) eines abgeleiteten eindimensionalen Drainagephasensättigungsprofils (20d), das aus dem mindestens einen gemessenen dreidimensionalen Drainagephasensättigungsprofil (25m) abgeleitet wurde, in den Maschinenlernalgorithmus (130);
Eingeben (S320) mindestens eines von einem gemessenen eindimensionalen Imbibitionsphasensättigungsprofil (30m) des Zielkernstopfens (50) in den Maschinenlernalgorithmus (130); und

Annähern (S330), unter Verwendung des Maschinenlernalgorithmus (130), der auf einem Prozessor (110) ausgeführt wird, des vorhergesagten dreidimensionalen Imbibitionsphasensättigungsprofils (35p) des porösen Gesteinsmediums (70) basierend auf der Eingabe.

9. Messsystem (100) zum Vorhersagen eines dreidimensionalen Imbibitionsphasensättigungsprofils (35p) zur Imbibition eines porösen Gesteinsmediums, wobei das System (100) umfasst:

eine Detektionsvorrichtung (150) zum Bestimmen von Daten eines Spezialkernanalyselabors (SCAL);
einen Prozessor (110) zum Ausführen eines Spezialkernanalyselabors (SCAL), eines Strömungssimulators (120) für poröse Medien und eines Maschinenlernalgorithmus (130);
einen Speicher (115), der mit dem Prozessor (110) verbunden ist, zum Speichern von Elementen von SCAL-Daten eines Zielkernstopfens (50) über einen Öl-Wasser-Kontaktwinkel (60) und optional eine relative Permeabilität (80) und/oder einen Kapillardruck (90); und
wobei der Maschinenlernalgorithmus nach einem der Ansprüche 1-7 trainiert wurde und konfiguriert ist, um das dreidimensionale Imbibitionsphasensättigungsprofil (35p) des porösen Gesteinsmediums nach Anspruch 8 vorherzusagen.

10. Messsystem (100) nach Anspruch 9, wobei:
die SCAL-Daten in Reservoirsimulationsmodellen für eine Vorhersage des Öl-und Gasfeldverhaltens verwendet werden.

11. Messsystem (100) nach den Ansprüchen 9 und 10, wobei:
die Detektionsvorrichtung (150) mindestens eines von einem linearen Röntgenscanner, einem Gammastrahlenscanner oder einer medizinischen CT-Kernflutungsausrüstung umfasst.

**Revendications**

1. Un procédé mis en œuvre par calculateur (5) pour l'entraînement d'un algorithme d'apprentissage machine (130) pour la prédiction d'un profil tridimensionnel de saturation de phase d'imbibition (35p) pour l'imbibition d'un milieu rocheux poreux (70), le procédé comprenant :

la dérivation (S140), à l'aide d'un processeur (110), d'un profil unidimensionnel de saturation de phase de drainage (20d) à partir d'un profil tridimensionnel de saturation de phase de drainage (25m) d'une carotte sélectionnée (50) du milieu rocheux poreux (70) ;
le calcul (S170), à l'aide du processeur (110) et d'un angle de contact huile-eau mesuré (60) dans la carotte sélectionnée (50) du milieu rocheux poreux (70), d'une pluralité de valeurs synthétiques pour une perméabilité relative (80) du milieu rocheux poreux (70) et d'une pression capillaire (90) du milieu rocheux poreux (70) ;
l'application (S190), à un simulateur de débit de fluide en milieu poreux (120), du profil tridimensionnel mesuré de saturation de phase de drainage (25m), du profil unidimensionnel dérivé de saturation de phase de drainage (20d), d'un profil unidimensionnel mesuré de saturation de phase d'imbibition (30m), de l'angle de contact huile-eau mesuré (60), des valeurs synthétiques calculées pour la perméabilité relative (80), des valeurs synthétiques calculées pour la pression capillaire (90), et d'un état d'hétérogénéité rocheuse (40) ;
la simulation (S200), à l'aide du simulateur de débit de fluide en milieu poreux (120), d'un profil tridimensionnel simulé de saturation de phase de drainage (25s) et d'un profil tridimensionnel simulé de saturation de phase d'imbibition (35s) ;
le calcul (S210), à l'aide du processeur (110), d'un profil unidimensionnel simulé de saturation de phase de drainage (20c) à partir du profil tridimensionnel simulé de saturation de phase de drainage (25s), et d'un profil unidimensionnel simulé de saturation de phase d'imbibition (30c) à partir du profil tridimensionnel simulé de saturation de phase d'imbibition (35s) ; et
l'entraînement d'un algorithme d'apprentissage machine (130) à l'aide du profil unidimensionnel simulé de saturation de phase de drainage (20s), du profil tridimensionnel simulé de saturation de phase de drainage (25s), du profil unidimensionnel simulé de saturation de phase d'imbibition (30s), et du profil tridimensionnel simulé de saturation de phase d'imbibition (35s).

2. Le procédé mis en œuvre par calculateur (5) de la revendication 1, comprenant en outre :
la mesure (S130) d'un profil tridimensionnel de saturation de phase de drainage (25m) à l'aide d'un outil de surveillance *in situ* de submersion de carotte comprenant un scanner tomodensitométrique.

3. Le procédé mis en œuvre par calculateur (5) des revendications 1 et 2, comprenant en outre :
la mesure (S210) de propriétés de fluide d'un pétrole brut.

**4.** Le procédé mis en œuvre par calculateur (5) des revendications 1 à 3, comprenant en outre :
l'identification (S120) d'une carotte jumelle (55) de la carotte sélectionnée (50).

**5.** Le procédé mis en œuvre par calculateur (5) de la revendication 4, comprenant en outre :
l'exécution (S150) d'une opération de vieillissement sur la carotte jumelle (55).

**6.** Le procédé mis en œuvre par calculateur (5) des revendications 1 à 5, comprenant en outre :
la mesure (S160) d'un angle de contact huile-eau (60) dans le milieu poreux (70) à l'aide d'au moins l'une d'entre une technique analytique ou une technique expérimentale.

**7.** Le procédé mis en œuvre par calculateur (5) des revendications 1 à 6, comprenant en outre :
la détermination d'un état d'hétérogénéité rocheuse (40) comprenant l'utilisation d'un scanner tomodensitométrique médical.

**8.** Un procédé mis en œuvre par calculateur (10) pour la prédiction d'un profil tridimensionnel de saturation de phase d'imbibition (35p) d'un milieu rocheux poreux (70), le procédé comprenant :

l'application (S300) à un algorithme d'apprentissage machine (130), qui a été entraîné conformément à l'une des revendications 1 à 7, d'au moins un profil tridimensionnel mesuré de saturation de phase de drainage (25m) d'une carotte cible (50) ;
l'application (S310) à l'algorithme d'apprentissage machine (130) d'un profil unidimensionnel de saturation de phase de drainage (20d) dérivé de l'au moins un profil tridimensionnel mesuré de saturation de phase de drainage ;
l'application (S320) à l'algorithme d'apprentissage machine (130) d'au moins l'un d'un profil unidimensionnel mesuré de saturation de phase d'imbibition (30m) de la carotte cible (50) ; et
l'approximation (S330), par exécution de l'algorithme d'apprentissage machine (130) sur un processeur (110), du profil tridimensionnel prédit de saturation de phase d'imbibition (35p) du milieu rocheux poreux (70) sur la base de ce qui a été appliqué.

**9.** Un système de mesure (100) pour la prédiction d'un profil tridimensionnel de saturation de phase d'imbibition (35p) pour l'imbibition d'un milieu rocheux poreux, le système (100) comprenant :

un dispositif de détection (150) pour la détermination de données d'analyse spéciale de carotte en laboratoire (SCAL) ;

un processeur (110) pour l'exécution d'une analyse spéciale de carotte en laboratoire (SCAL), un simulateur de débit de fluide en milieu poreux (120) et un algorithme d'apprentissage machine (130) ;
une mémoire (115), reliée au processeur (110), pour le stockage de rubrique de données SCAL d'une carotte cible (50) sur un angle de contact huile-eau (60), et éventuellement d'une perméabilité relative (80), et/ou d'une pression capillaire (90) ; et
dans lequel l'algorithme d'apprentissage machine a été entraîné conformément à l'une des revendications 1 à 7 et est configuré pour prédire conformément à la revendication 8 le profil tridimensionnel de saturation de phase d'imbibition (35p) du milieu rocheux poreux.

**10.** Le système de mesure (100) de la revendication 9, dans lequel :
les données SCAL sont utilisées dans des modèles de simulation de gisement pour la prédiction du comportement d'un champ pétrolifère ou gazier.

**11.** Le système de mesure (100) des revendications 9 et 10, dans lequel :
le dispositif de détection (150) comprend au moins l'un d'entre un scanner à rayons X, un scanner à rayons gamma, ou un équipement tomodensitométrique médical de submersion de carotte.

100

| 120 | | 5 |
| 130 | 110 | 10 |

115   150

FIG. 1A

FIG. 1B

```
                        ┌──────────┐
                        │  START   │                          ╱ 5
                        └──────────┘                       ◄──
                             │
                             ▼
S100          ┌─────────────────────────────────────┐
              │  Measure rock properties            │
              └─────────────────────────────────────┘
                             │
                             ▼
S110          ┌─────────────────────────────────────┐
              │  Measure fluid properties of the crude oil │
              └─────────────────────────────────────┘
                             │
                             ▼
S120          ┌─────────────────────────────────────┐
              │  Identify sister plug (55) for the selected core │
              │  plug (50)                          │
              └─────────────────────────────────────┘
                             │
                             ▼
S125          ┌─────────────────────────────────────┐
              │  Determine rock heterogeneity state (40) │
              └─────────────────────────────────────┘
                             │
                             ▼
S130          ┌─────────────────────────────────────┐
              │  Measuring three-dimensional drainage phase │
              │  saturation profile (25m)           │
              └─────────────────────────────────────┘
                             │
                             ▼
S140          ┌─────────────────────────────────────┐
              │  Deriving one-dimensional drainage phase │
              │  saturation profile (20d)           │
              └─────────────────────────────────────┘
                             │
                             ▼
S150          ┌─────────────────────────────────────┐
              │  Performing ageing operation        │
              └─────────────────────────────────────┘
                             │
                             ▼
S160          ┌─────────────────────────────────────┐
              │  Measuring oil-water contact angle (60) in the │
              │  porous medium (70)                 │
              └─────────────────────────────────────┘
                             │
                             ▼
S170          ┌─────────────────────────────────────┐
              │  Calculating plurality of synthetic values for a │
              │  relative permeability (80) and a capillary │
              │  pressure (90) of the core plug (50) │
              └─────────────────────────────────────┘
                             │
                             ▼
                             ●
                             A
```

FIG. 1C

● A

5

S190

Feeding
- measured three-dimensional drainage phase saturation profile (25m),
- derived one-dimensional drainage phase saturation profile (20d),
- one-dimensional imbibition phase saturation profile (30m),
- oil-water contact angle (60),
- calculated relative permeability (80),
- calculated capillary pressure (90), and
- rock heterogeneity state (40)
into SCAL-simulator (120)

S200

Simulating simulated three-dimensional drainage phase saturation profile (25s) and simulated three-dimensional imbibition phase saturation profile (35s)

S210

Calculating simulated one-dimensional drainage phase saturation profile (20s) and simulated one-dimensional imbibition phase saturation profile (30s)

S220

Train machine learning algorithm (130)

END

Fig. 1D

Core length

Average phase saturation

Fig. 1E

Fig. 2A

Fig. 2B

Fig. 2C

Fig. 2D

Permeability I (PERMX)
Permeability I [mD]

1000.0000

100.0000

10.0000

1.0000

0.1000

Train &
Validate

Blind Test

Fig. 3

Fig. 4

Fig. 5

Fig. 6A

Fig. 6B

FIG. 7

10

```
          ┌─────────────┐
          │    START    │
          └─────────────┘
                 │
                 ▼
      ┌──────────────────────────────┐
      │ Inputting into trained       │
S300  │ machine learning             │
      │ algorithm (130) measured     │
      │ three-dimensional            │
      │ drainage phase saturation    │
      │ profile (25m)                │
      └──────────────────────────────┘
                 │
                 ▼
      ┌──────────────────────────────┐
      │ Inputting into trained       │
S310  │ machine learning             │
      │ algorithm (130) derived      │
      │ one-dimensional              │
      │ drainage phase saturation    │
      │ profile (20d)                │
      └──────────────────────────────┘
                 │
                 ▼
      ┌──────────────────────────────┐
      │ Inputting into trained       │
S320  │ machine learning             │
      │ algorithm (130) measured     │
      │ one-dimensional              │
      │ imbibition phase saturation  │
      │ profile (30m)                │
      └──────────────────────────────┘
                 │
                 ▼
      ┌──────────────────────────────┐
      │ Approximating predicted      │
S330  │ three-dimensional            │
      │ imbibition phase saturation  │
      │ profile (35p) using          │
      │ trained learning algorithm   │
      │ (130)                        │
      └──────────────────────────────┘
                 │
                 ▼
          ┌─────────────┐
          │     END     │
          └─────────────┘
```

## REFERENCES CITED IN THE DESCRIPTION

### Patent documents cited in the description

- WO 2019210102 A1, Dogru
- US 10718188 B2, Dinariev
- US 10198535 B2, Li
- US 20180121579 A1

### Non-patent literature cited in the description

- **MOTEALLEH SIYAVASH ; ASHOURIPASHAKI MANDANA ; DICARLO DAVID ; BRYANT STEVEN.** Model of Drainage and Imbition in 3D Fractionally Wet Porous Media. *Transport in Porous Media,* 2013, vol. 99 (3), 581-611

- **JAMSHIDIAN MAJID ; MANSOURI ZADEH MOSTAFA ; HADIAN MOHSEN ; MOGHADASI RAMIN ; MOHAMMADZADEH OMID.** A novel estimation method for capillary pressure curves based on routine core analysis data using artificial neural networks optimized by Cuckoo algorithm - A case study. *Fuel,* 2018, vol. 220, 363-378
- **FENWICK DARRYL H ; BLUNT MARTIN J.** Three-dimensional modeling of three phase imbibition and drainage. *Advances in Water Resources,* 1998, vol. 21 (2), 121-143